# EUROPEAN PATENT APPLICATION

(11) **EP 2 772 253 A1**
(43) Date of publication of application: **03.09.2014**
(21) Application number: 13157034.3
(22) Date of filing: 27.02.2013
(51) Int. Cl.: A61K 31/245, A61P 23/02, A61C 19/06

(54) **Topical formulations useful for treating gagging reflex**

(71) Applicant: Lenares, Antonio, 6925 Gentilino (CH)
(72) Inventor: Lenares, Antonio, 6925 Gentilino (CH)
(74) Representative: Zardi, Marco

(57) **Abstract**

New formulations are disclosed, based on highly concentrated benzocain in a semisolid system, suitable for topical administration within selected areas of the mouth, having a strong and consistent efficacy in reducing/eliminating the gagging reflex in patients exposed to it, especially those undergoing dental manipulations.

## Description

### STATE OF THE ART

Dental procedures and dental hygiene procedures usually have a high impact on the patient, both from the physical and psychological point of view. The use of local anaesthetics, although useful for reducing or eliminating the immediate pain caused by the dental manipulation, does not eliminate the general discomfort and the status of anxiety suffered by many patients during dental sessions. These procedures often involve touching remote areas of the mouth with foreign bodies like gloved fingers, mechanical appliances, strong-tasting liquids, pastes etc.

A number of patients react to these inputs with uncontrollable gagging, i.e. spasms of the larynx urging him/her to constrict the throat, in an effort to expel foreign bodies from the mouth, the spasm sometimes extends to the neck and other parts of the body causing uncontrolled movements of the patient: this may provoke a sudden halt of the manipulation by the dentist, which is sometimes dangerous, depending on the content of the mouth and the stage of treatment when gagging occurs.

Gagging is a vago-vagal reflex of the sympathetic nervous system, highly subjective, and quite hard to address and inhibit. As mentioned above, topical anaesthetic solutions normally used by the dentist to reduce pain, are not capable to obtain a significant inhibition of the gagging reflex; normal antiemetic agents are totally ineffective.

For these reasons there is still an outstanding need for treatments and medicaments capable of strongly reduce or eliminate the gagging reflex. These should increase the general comfort during a dental session, both for the patient and dentist, reduce the impact of the necessary manipulations, reduce the time to complete them, and improve general efficiency.

### SUMMARY OF THE INVENTION

The present Applicant has unexpectedly found that a particular formulation of benzocaine, in a semisolid topical carrier, suitable for in situ application to specific areas of the buccal mucosa, strongly or totally eliminates the gagging reflex, even in most sensitive patients, allowing them to undergo and complete a dental session in total comfort.

### DESCRIPTION OF THE FIGURES

Figure 1: Preferred administration areas for the formulation of the invention (A, B: areas of application)
Figure 2: Applicator according to the invention, in the form of a rod, with drug-loaded ends according to a first embodiment; the formulation of the invention may be coated onto and/or impregnated into the applicator ends
Figure 3: Applicator according to the invention, in the form of a rod, with drug-loaded ends according to a second embodiment; the formulation of the invention may be coated onto and/or impregnated into the applicator ends, or added into in the concave area formed by each end.

### DESCRIPTION OF THE INVENTION

A first object of the invention is a dental formulation comprising benzocaine, in a semisolid carrier, suitable for a topical administration on a buccal mucosa, for use in preventing or treating gagging in a patient in need thereof. The dental formulation comprises benzocaine at a high concentration, typically between 15 and 25% by weight, preferably between 18 and 22%, more preferably about 20%, in association with a semisolid carrier for topical administration on the mucosa. The dental formulation is more concentrated than the injectable compositions of local anaesthetics used for dental anaesthesy; it also differs therefrom in that it comprises the semisolid carrier, which is evidently unsuitable for injections.

The dental formulation of the present invention may be in particular the form of creams, ointments, gels, pastes and similar forms, comprising a semi-solid carrier. For the purpose of the invention, the term "semi-solid carrier" means the overall content of said creams, ointments, gels, pastes, except the benzocaine.The carrier can be conveniently selected by compounding ingredients well-known in the art, according to the following non-limitative list.

Creams. oily bases that can be used for creams are fatty alcohols, e.g. C12 to C18 alcohols, such as lauryl, cetyl or stearyl alcohol, fatty acids, especially those containing from C10 to C18, such as palmitic or stearic acid, fatty acid esters, e.g. glyceryl tricaprilocaprate (neutral oil) or cetyl palmitate, waxes, for example isopropyl myristate, wool wax or beeswax, and/or hydrocarbons, semi-solid or solid substances or mixtures thereof, for example petroleum jelly (petrolatum, Vaseline) or paraffin oil. Suitable emulsifiers can be present in the formulation: for example fatty acid esters of polyalcohols and/or ethylene oxide adducts thereof, glycerol fatty acid esters or partial fatty acid esters of polyhydroxyethylene sorbitan, such as polyglycerol fatty acid esters or polyoxyethylene sorbitan fatty acid esters (Tweens), and also polyoxyethylene fatty alcohol ethers or fatty acid esters (for example Tagat S), or corresponding ionic emulsifiers, such as alkali metal salts of fatty alcohol sulfates, for example sodium lauryl sulfate, sodium cetyl sulfate or sodium stearyl sulfate. Further additives can be for example humectants, such as polyalcohols, such as glycerol, sorbitol, propylene glycol and/or polyethylene glycols, preservatives, perfumes, gelling agents, etc.

Ointments. suitable for the fatty phase are especially hydrocarbons, for example petroleum jelly, paraffin oil and/or hard paraffins, possibly mixed with hydroxy compounds, such as fatty alcohols or esters thereof, for example cetyl alcohol or wool wax alcohols, or wool wax or beeswax. Further useful lipohilic ingredients are natural or partially synthetic fat, such as fatty acid esters of glycerol, for example coconut fatty acid triglyceride, or preferably hardened oils, for example hydrogenated groundnut oil, castor oil or waxes, also fatty acid partial esters of glycerol, for example glycerol mono- and di-stearate, and also, for example, the fatty alcohols increasing the water-absorption capacity, emulsifiers and/or additives mentioned in connection with the ointments. Emulsifiers are corresponding lipophilic substances, for example of the type indicated above, such as sorbitan fatty acid esters (Spans), for example sorbitan oleate and/or sorbitan isostearate. Additives to the aqueous phase are, inter alia humectants, such as polyalcohols, for example glycerol, propylene glycol, sorbitol and/or polyethylene glycol, and also preservatives, perfumes, etc.

Gels components can be high molecular weight inorganic components having gel-forming properties, such as aluminium silicates, for example bentonite, magnesium aluminium silicates, or colloidal silicic acid, polysaccharides such as celluloses, starches, tragacanth, gum arabic and agar-agar, and gelatin, alginic acid and salts thereof, lower alkylcelluloses, for example methyl- or ethylcellulose, carboxy- or hydroxy-lower alkylcelluloses, for example carboxymethyl-or hydroxyethyl-cellulose, polyvinyl alcohol, polyvinylpyrrolidine, polyacrylates or polymethacrylates.

The dental formulations according to the invention may also comprise conventional additives and adjuvants for topical applications, such as preservatives, especially paraben esters like methylparaben, ethylparaben, propylparaben, butylparaben, or quaternary ammonium compounds like benzalkonium chloride, or formaldehyde donors like imidazonidinyl urea, or alcohols like benzyl alcohol, phenoxyethanol or acids like benzoic acid, sorbic acid; acids or bases used as pH buffer excipients; antioxidants, especially phenolic antioxidants like hydroquinone, tocopherol and derivatives thereof, as well as flavonoids, or miscellaneous antioxidants like ascorbic acid,ascorbyl paimitat ; perfumes; fillers such as kaolin or starch; pigments or colorants ; UV-screening agents; moisturizers, especially glycerin, butylen glycol, hexylen glycol, urea, hyaluronic acid or derivatives thereof; anti-free radical agents such as vitamin E or derivatives thereof; penetration enhancers especially propylene glycol; ethanol; isopropanol; dimethylsulfoxide; N-methyl-2- pyrrolidone; fatty acids/alcohols such as oleic acid, oleyl alcohol; terpenes such as limonen, menthol, 1-8 cineole; alkyl esters such as ethyl acetate, butyl acetate; ion pairing agents such as salicylic acid, flavoring agents, etc. Further details concerning suitable topical formulations may be obtained by reference to standard textbooks such as Banker and Rhodes (Ed) Modern Pharmaceutics 4th ed. (2002) published by Marcel Dekker Inc.; Harry's Cosmeticology (2000), 8th Edition, Chemical Publishing Co.; Remington's Pharmaceutical Sciences 20th ed Mack Publishing Co.(2000).

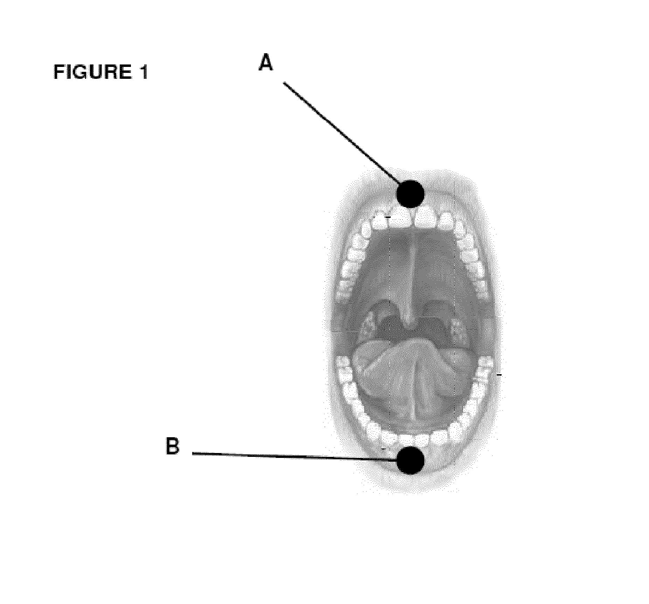

The dental formulation of the invention, even if washed out (after the required minimum residence time of 10 seconds), maintains its effect on the patient for about 1.5 hours after application, thus allowing the dentist ample time to complete standard or even complicated dental procedures. Residence times longer than 10 second result in longer-lasting protection. The anti-gagging effect is particularly strong and long-lasting when the formulation is applied to an upper and a lower areas of the buccal mucosa, in particular the vestibular area located between the upper incisors and the upper lip, and the vestibular area located between the lower incisors and the lower lip (cf. marked areas in Figure 1); the semisolid structure of the formulation makes it suitable for permanence in these areas for the required residence time or more, thereby maximizing its effect.

The dental formulation is conveniently administered at the beginning of a dental session, i.e. prior to any manipulations, thus ensuring a strong and extended effect over the entire dental session; however it may also be administered during an ongoing session to treat unexpected gagging episodes. In all circumstances, the simplicity of access of the most sensible vestibular areas referred above, and the semisolid structure of the formulation, allows a quick administration by the dentist or even the patient himself: in fact the resulting application procedure is simple and straightforward, not requiring at all medical, paramedical or dental skills. It is accordingly possible for the patient to self-administer the medication at home, before going to the dentist, without even informing the dentist.

The treatment is preferably of preventive type so as to avoid gagging to occur at all; however, if needed, it can also be used in prophylactic manner on an ongoing gagging reflex: in this case, gagging will be strongly reduced or eliminated and future gagging will remain inhibited during the time window of efficacy of the formulation.

The present invention includes the use of said formulation to prevent or treat gagging reflex in a patient requiring it, as well as the formulation for such use.

The invention further includes the above described dental formulation as a product, being suitable for application to selected areas of the mouth, as above described.

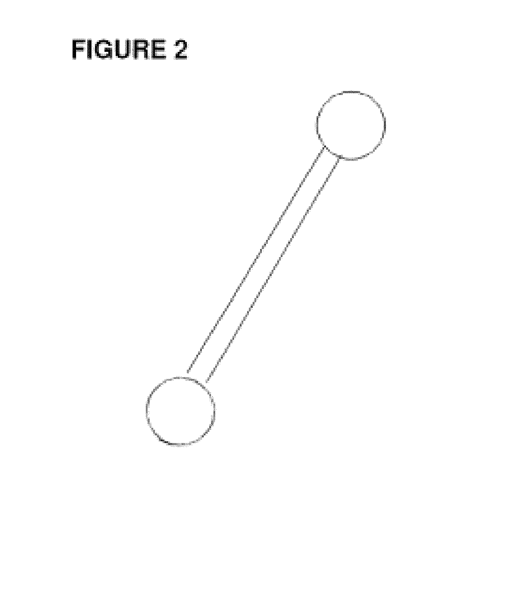

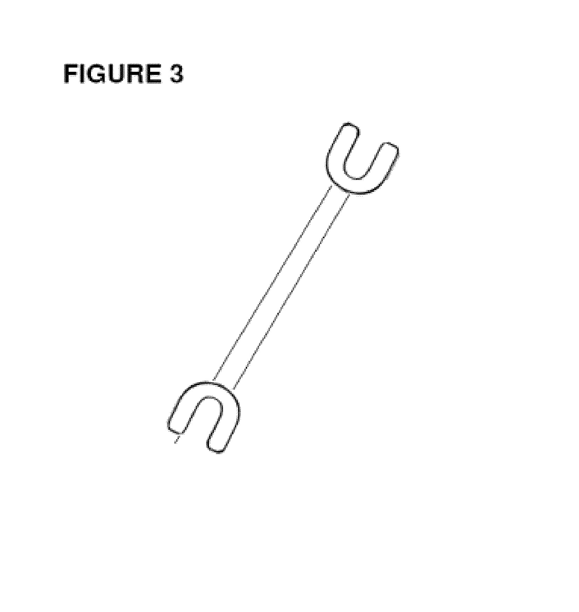

Finally the invention includes an applicator, especially adapted for topically delivering the present formulation in the mouth, suited for contacting an upper and a lower area of the mouth. The applicator is an elongated body, typically a rod. The upper and lower ends of the applicator are preventively loaded with a suitable amount of the formulation above described. The ends are preferably shaped in a form/structure suitable to incorporate the required amount of formulation, e.g. they can have recesses or protrusions, tissues, sponges and the like, containing the formulation; in one embodiment the ends are made, partially or totally of cotton impregnated or coated with the formulation. The applicator has a length allowing it to contact, respectively with its upper and lower ends, an upper and a lower area of the mouth; a non limitative range of length (exclusive of the formulation-loaded end areas) comprised between 2 and 10 cm, preferably between 3 and 9 cm, or more preferably between 4 and 6 cm. The loaded ends, meaning with this term the terminal potions of the applicator which contain the medication, have preferably length, height and depth of about 6-12 mm, more preferably about 9 mm. The applicator is typically contained in a sealed package which can be opened at the moment of use. Upon use, the patient, dentist or any other person on their behalf, takes the applicator, preferably a rod, guides it in proximity of or inside the mouth of the patient, and contacts the loaded ends with the upper and lower parts of the mouth, typically the vestibular area between the upper incisors and the vestibular area between the lower incisors. The applicator is maintained in this position for at least 10 seconds, after which the formulation is delivered and the applicator can be disposed. A non-limitative realization of the applicator is shown in Figures 2 and 3 in the form of a rod, with different types of loaded ends.

A further object of the invention is a dental kit comprising: (a) the dental applicator described above and (b) the dental formulation described above within a suitable container. In this case, the applicator is not preventively loaded; loading is accomplished upon use by contacting the applicator ends with the formulation.

Finally, the invention includes a process to manufacture the above formulation per se comprising admixing benzocaine with an adequate amount of semi-solid carrier for topical use. The invention also includes a process to manufacture the applicator and/or kit described above.

### EXAMPLE

A total of 80 patients, with a known history of gagging reflex secondary to dental procedures, were administered topically with a cream containing 20% wt of benzocaine. The formulation was topically applied onto two areas of the mouth between the central incisors, respectively in its upper and lower part (Fig.1). The patients were then subjected to scheduled dental interventions and/or dental hygiene interventions, involving the necessary manipulations within the mouth. The medication proved effective almost immediately, as demonstrated by the fact that the intervention could be started 10-30 seconds after administration, without reporting any gagging; the interventions lasted from about 20 to about 70 minutes: no gagging was experienced by the tested patients throughout this entire period, and the interventions could be completed in standard conditions, with no need for special precautions.

## Claims

1. A dental formulation comprising benzocaine, at a concentration between 15 and 25% by weight in a semisolid carrier, suitable for a topical administration on a buccal mucosa, for use in preventing or treating gagging in a patient in need thereof.

2. A dental formulation according to claim 1, where the concentration of benzocaine is between 18% and 22%.

3. A dental formulation according to claims 1-2, where the concentration of benzocaine is about 20%.

4. A dental formulation according to claims 1-3, for administration to a patient undergoing a dental procedure.

5. A dental formulation according to claims 1-4, in form of gel, cream, ointment, or paste.

6. A dental formulation according to claims 1-5, suitable to adhere to the site of administration in the mouth for 10 seconds of more after administration.

7. A dental formulation according to claims 1-6, which is effective on gagging for at least 1.5 hour after administration.

8. A dental formulation according to claims 1-7, inclusive of instructions and/or means for its application on at least one upper area and at least one lower area of the mouth.

9. A dental kit including the formulation according to claims 1-8 and an applicator for application of the formulation on at least one upper area and at least one lower area of the mouth.

10. The dental kit according to claim 9, where the applicator comprises an elongated body having upper and lower ends loaded with a suitable amount of said formulation.

11. A dental applicator for administering the formulation of claims 1-8, comprising an elongated body having upper and lower ends loaded with a suitable amount of said formulation.

12. The dental applicator according to claim 11, having length comprised between 2 and 10 cm.

13. The dental applicator according to claims 11-12, in which said ends have length, height and depth comprised between 6 and 12 mm.
